# EUROPEAN PATENT SPECIFICATION

(11) **EP 1 024 778 B1**
(45) Date of publication and mention of the grant of the patent: **17.09.2003**
(21) Application number: 98945945.8
(22) Date of filing: 08.09.1998
(51) Int. Cl.: A61K 6/083, A61K 6/02

(54) **PROTECTIVE VARNISH FOR DENTIN**
DENTINSCHUTZLACK
VERNIS PROTECTEUR DE LA DENTINE

(30) Priority: 22.10.1997 US 955902
(43) Date of publication of application: 09.08.2000
(73) Proprietor: DENTSPLY INTERNATIONAL, INC., York, PA 17405-0872 (US)
(72) Inventor: PFLUG, Kai, D-78467 Konstanz (DE); LYNCH, Edward, London SE21 8BD (GB)
(74) Representative: Wächtershäuser, Günter, Prof. Dr.
(86) International application number: US9818692
(87) International publication number: WO99020227

(56) References cited:
- EP-A- 0 282 633
- WO-A-89/10113
- US-A- 4 966 934
- DATABASE WPI Derwent Publications Ltd., London, GB; AN 88-253528 XP002097599 KANEBO LTD: "Light curable resin compsn. for coating plastic prod.- contains dipentaerythritol acrylate monomer, volatile solvent and polymerisation initiator" & JP 63 183904 A
- DATABASE WPI Derwent Publications Ltd., London, GB; AN 93-040676 XP002097600 KANEBO LTD. : "Resin compsn. polymerisable with visible light- contains polyfunctional acrylate monomer, volatile solvent and thioxanthone-contg. photopolymerization catalyst " & JP 04 366113 A
- DATABASE WPI Derwent Publications Ltd., London, GB; AN 93-040675 XP002097601 KANEBO LTD: "Resin compsn. polymerisable by visible light irradiation contains polyfunctional acrylate monomer, volatile solvent and photopolymerization catalyst contg. alpha-aminoacetone " & JP 04 366112 A
- DATABASE WPI Derwent Publications Ltd., London, GB; AN 92-127233 XP002097602 KANEBO LTD: "Visible photopolymerization curable type compsn. for dental materials-contains caprolactone modified-dipentaerythritol hexaacrylate monomer, volatile solvent and polymerisation initiator " & JP 04 069311 A
- IMAZATO S ET AL: "Antibacterial effect of composite incorporating Triclosan against Streptococcus mutans" JOURNAL OF OSAKA UNIVERSITY DENTAL SCHOOL, XP002074014

## Description

### Technical Field

The invention relates to a protective varnish for dentin. This varnish contains polymerizable resins. It may also contain an antimicrobial agent. Furthermore, it may contain fillers.

The varnish is effective in protecting the dentin from mechanical forces as after curing it forms a polymeric network that reinforces and mechanically strengthens the dentin. It also reduces hypersensitivity by blocking dentin tubules.

The protective varnish may contain an antimicrobial agent. This particular varnish shows an antimicrobial effect after curing. The antimicrobial effect is achieved by incorporation of a broad spectrum antimicrobial agent such as 2,4,4'-trichloro-2'-hydroxydiphenyl ether which is also known as triclosan.

### Background

The relationship between bacterial flora and the development of periodontal disease and caries has been shown (P. Axelsson et al in: J. Clin Perio 5, 133-151 (1978)]. To achieve reduction of these dental diseases it is necessary to control the bacterial flora. The most widely used approach to date to control the bacterial flora in the oral cavity has been mechanical cleaning methods, such as brushing the teeth. Although this method has proved to be fairly successful in treating individuals, there is still a high recurrence rate. There is also the problem of motivating people to good oral hygiene habits that they will maintain throughout their lives.

A variety of materials have been developed to suppress oral microorganisms. These include mouth rinses, dentifrices and gels containing antibacterial agents such as chlorhexidine and quarternary ammonium salts. These materials only offer a short-term antimicrobial effect. Sustained release of an antimicrobial agent has been achieved by embedding chlorhexidine in a polymer to form a varnish. However, the materials developed so far display some disadvantages. Reported side effects of chlorhexidine, including staining and altered taste perception, have limited its acceptance. Attempts to reduce these side effects by using dilute solutions and flavoring agents have only been partly successful.

More importantly, these chlorhexidine varnishes are only effective for a limited period of time because the uncrosslinked polymer matrix does not prevent the antimicrobial agent from leaching out within a few days. For example, U.S. Pat. No. 4,496,322 discloses a dental varnish which contains chlorhexidine acetate, a benzoin gum, and an orally acceptable solvent. The composition, once applied to the teeth, is allowed to dry and gives a film which provides sustained release of the antimicrobial agent over a period of a few days. PCT WO 89/10736 describes dental glass polyalkenoate cements made soluble in oral fluids by the addition of chlorhexidine. However, these materials dissolve after 1-4 weeks on the teeth and therefore are not suitable as long-term dental material.

The broad spectrum antimicrobial agent triclosan has been known for more than 25 years. It has been used extensively in soaps, hand disinfectants, deodorants, laundry products, textile treatment, detergents, foot powder, shampoos and disposable paper products. It is soluble in many organic solvents, stable to hydrolysis and is regarded as safe for human contact and the environment. Triclosan is a highly effective antimicrobial agent with a broad spectrum of activity against both Gram-positive and Gram-negative bacteria as well as fungi, yeasts and viruses [Ciba-Geigy: Irgasan MP: General information on chemical, physical, microbiological and toxicological properties]. In long-term experiments, no development of bacterial resistance to triclosan was found [C. L. Jones et al., in: J. Dent Res. 67, 46-50 (1988)].

More recently triclosan has also started to be used in oral care products, e.g. toothpastes and mouth rinses. The Colgate Palmolive Company has employed triclosan as a toothpaste ingredient that has been proven to be effective against plaque bacteria [Bolden T. E. et al. in: J. Clin. Dent. 4, 125-131 (1992)]. Dentifrices containing triclosan have been tested and found to reduce plaque [K. W. Stephen et. al., in: J. Periodontol. 61, 674-679 (1990)].

A number of trends have emerged in dentistry recently. The number of elderly patients is increasing; also, the number of teeth per patient will increase. The number of recessions in the younger generation will increase due to wrong and too intense use of tooth brushes. As a consequence of these trends, the number of exposed dentin surfaces will increase due to periodontal diseases and artificial lesions. Exposed dentin causes a number of problems, such as patient discomfort due to hypersensitivity. Further, exposed areas of dentin are less resistant against attacks by acids. This results in a higher risk of caries in these exposed areas.

For enamel, pit and fissure sealants are commonly used to help prevent occlusal caries. These sealants are applied to the tooth surface to fill and seal pits and fissures by forming a thin plastic coating. When used in regular professional care programs of preventive dentistry, the sealants have the potential to eliminate caries [Simonsen, in: JADA, Vol. 122, October 1991; Wendt, Koch, in: Swed. Dental Journal, 12, 180-185 (1988)].

An equivalent procedure for exposed dentin has not been known in the industry. Products currently available concentrate on hypersensitivity prevention (Gluma Desensitizer, heraeus Kulzer Inc.; MS-Coat, J. Morita Europe GmbH) or combine this with an antimicrobial effect (Cervitec, Ivoclar Viviadent). None of the products available offers a long-term antimicrobial effect or a mechanical protection of the exposed dentin.

### Summary of the invention

It is therefore an object of the invention to provide a protective varnish for exposed dentin. This is desirable as exposed dentin may cause hypersensitivity and is less resistant against acid attacks, and therefore presents a higher risk of caries.

In general, a protective vanish for dentin comprises a matrix of polymerizable resins in a solvent that is capable of penetrating deep into the dentin. After curing, the polymer matrix formed reinforces the dentin, making it more abrasion-resistant. Furthermore, the polymer formed blocks dentin tubules, reducing hypersensitivity of the dentin. The protective varnish also comprises an antimicrobial agent. In such a formulation, the polymer matrix formed after curing prevents the antimicrobial agent from leaching out rapidly.

### Detailed description of the invention

The present invention provides a protective varnish for exposed dentin. The varnish has an antimicrobial effect by incorporation of an antimicrobial agent, triclosan. A preferred range of triclosan is from about 0.001 to about 20 percent by weight based upon 100 percent by weight of the varnish. The triclosan is added in the unpolymerized state of the varnish.

From about 15 to about 85 percent by weight of polymerizable materials are provided to form the polymer network. Useful polymerizable materials include methacrylate and acrylate monomers having at least one unsaturated double bond, and mixtures thereof. Preferred polymerizable monomers are those that are light-curable.

Useful solvents in the varnish include water, acetone, ethanol, ethyl acetate, other organic solvents with boiling points below that of water, and mixtures thereof. A useful amount of solvent would be from about 15 to about 85 percent by weight based upon 100 percent by weight of the varnish.

Other varnish components may include resins, fillers, fluoride, stabilizers, initiators, solvents and other substances commonly used in dental materials.

After curing, a polymeric network is formed that serves as a matrix for the triclosan, thereby preventing rapid leaching. This polymeric network ensures the long-term antimicrobial efficacy of the triclosan.

The varnish comprises polymerizable monomers in a solution of low viscosity. This composition allows deep penetration of the dentin, resulting in filling of the dental tubule and displacement of water in the dentin. After curing, the monomers polymerize to form a crosslinked polymeric network. The polymer is formed within the dentin as well as on top of it. The combined effect of the strengthening of the dentin from within and the polymeric layer on top of the dentin offer mechanical protection of the dentin, making it more resistant to abrasion. This protection may be enforced by incorporating filler particles into the varnish.

Examples of useful filler materials include ground glass or quartz, highly dispersed silica, zeolite, laponite, kaolinite, vermiculite, mica, ceramic metal oxides, alumina, pyrogenic silica, sparingly volatile oxides of titanium, zirconium, germanium, tin, zinc, iron chromium, vanadium, tantalum, niobium, and mixtures thereof. Preferably, these materials are used as very fine particles, most preferably with a primary particle size of about 1 nm to about 100 nm. These fillers of a primary particle size of about 1 nm to about 100 nm will be referred to as nanofillers herein. A range of useful filler material content in the varnish is from about 1 to about 15 percent by weight. The varnishes according to the invention preferably have a viscosity of from about 0.0001 to about 1 Pas. Further, it is preferably coated to dentin in a thickness of from about 0.01 to about 1000 micrometers.

One preferred filler is treated with an agent enabling the filler to form a stable sol in an organic solution with a viscosity below about 1 Pas. Silanating agents are preferred, and it is further preferred to treat the filler before formation of the sol. Sol formation may be facilitated by employing high shear forces, for example, by employing sonication or ultraturrax treatment.

Preferred silanating agents include those having at least one polymerizable double bond and at least one group that easily hydrolyses with water. Examples of such agents include 3-methacryloxypropyltrimethoxysilane, 3-methacryloxypropyldimethoxy-monochlorosilane, 3-methacryloxypropyldichloromonomethoxylsilane, 3-methacryloxypropyltrichlorosilane, 3-methacryloxypropyldichloromonomethyl-silane, 3-methacryloxypropylmonochlorodimethylsilane, and mixtures thereof.

Apart form mechanical protection, the varnish reduces dentin hypersensitivity by blocking dentin tubules, thus blocking the pulp from thermal, mechanical or chemical stimuli.

### Examples

The following examples are given to illustrate the present invention without attempting to limit the invention.

### Example 1: Antimicrobial Protective Varnish Containing Triclosan

An antimicrobial protective varnish for exposed dentin was prepared containing the following components (wt% is weight percent):
- 80 wt% ethanol
- 10.5 wt% UDMA-resin (2,7,7,9,15-pentamethyl-4, 13-dioxa-5,12-diazahexadecan-1, 16-diyldimethacrylate)
- 3.0 wt% urethane resin R5-62-1 (7,7,9,63,63,65-Hexamethyl-4,13,60,69-tetraoxo-3,14,19,24,29,34,39,44,49,54,59,70-dodecanoxa-5,12,61,68-tetraaza-doheptaconta-1,72-diyldimethacrylate)
- 0.6 wt% ethyl 4-dimethylaminobenzoate
- 0.1 wt% 2,6-di-tert-butyl-p-cresol
- 0.2 wt% cetylamine hydrofluoride
- 0.6 wt% trimethylolpropane trimethacrylate
- 0.2 wt% camphorquinone.
- 4.8 wt% PENTA (dipentaerythritol penta acrylate monophosphate)

To this mixture (100 wt%), triclosan was added in various concentrations, as will be shown below.

This varnish has a low viscosity and deeply penetrates the dentin. After application, the ethanol solvent is removed by air-drying. Curing is done with a dental curing lamp with visible light for 20 seconds. A thin, strong polymeric film (thickness approximately 2-6 micrometers) remains.

### Antimicrobial tests

In-vitro tests showed a film of the composition above (2 wt% triclosan) to have an antimicrobial effect on streptococcus mutans as follows:

Test plates were filled with approximately 50 microliters of an antimicrobial varnish composition comprising the substances above. As a reference, similar formulations were prepared not containing fluoride and/or triclosan but with an otherwise unchanged composition. The solvent ethanol was evaporated under nitrogen and the varnish was light cured under nitrogen to prevent incomplete polymerization due to oxygen inhibition.

These test plates were filled with 50 microliters of a liquid containing approximately 5 X 10⁴ CFU of streptococcus mutans in PBS + 10% serum. Contact time was 30 seconds, 10 minutes (min.) 1 hour (h), 3 hours and 6 hours at 37° C. An unfilled test plate was used as negative control. Each test was run three times. Subsequently the test solution was transferred to a new plate and subjected to enrichment. An MTT test was carried out to detect living streptococci mutans.

This test was run on two different days. The tables show the results obtained.

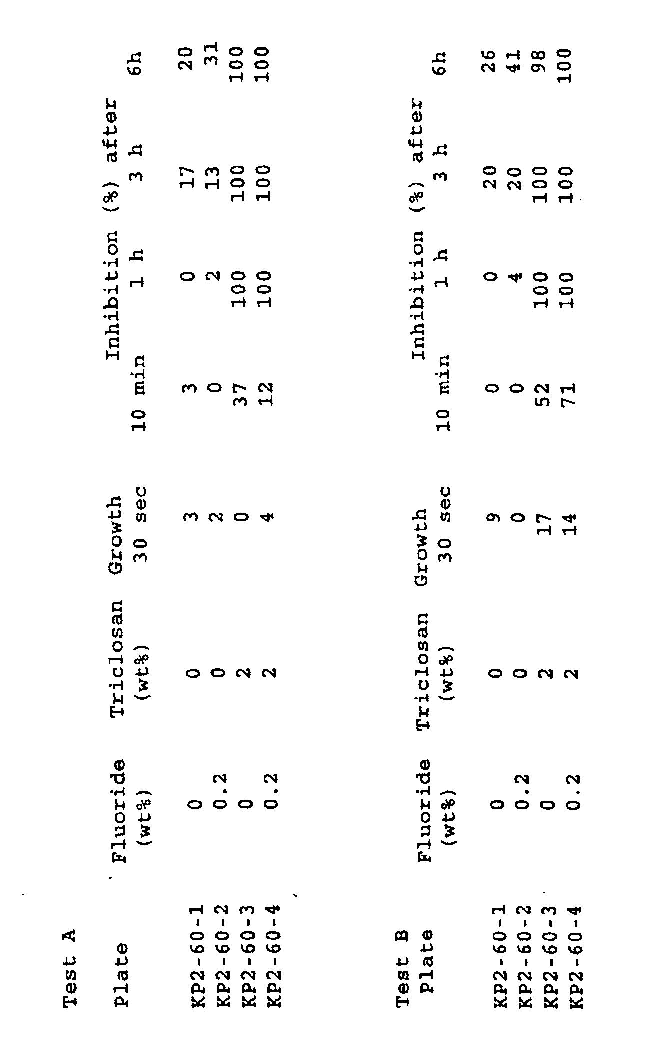

These tests show that the antimicrobial varnish formulations containing triclosan have a high efficacy with regard to effect on streptococcus mutans.

To show that an antimicrobial effect is still present after elution of the material, the test was repeated with the same test plates after pre-elution in 0.9% NaCl for 7 days at 37°C. Though the antimicrobial efficacy is somewhat lower, it still is significant in the triclosan-containing test plates.

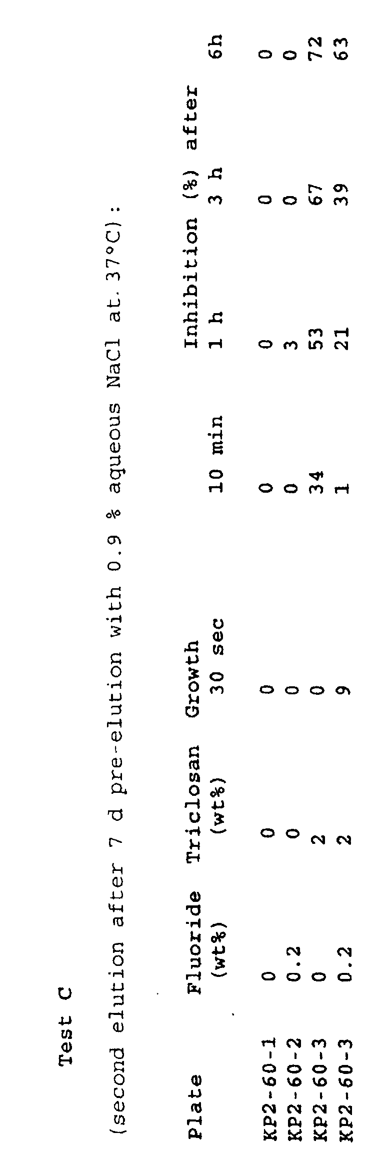

### Leaching tests

To demonstrate the low leaching rate of triclosan despite its antimicrobial efficacy in the varnish, plaques of approximately 1.2 g (width 2 mm, diameter 24 mm) were made from a mixture of the varnish components except for the solvent ethanol (triclosan content 6.25 wt% based on resin mixture).

These plaques were light-cured and stored in artificial saliva (Ringer solution) for 20 days at 37°C. By UV/Vis spectroscopy, no triclosan could be found in the artificial saliva. Control experiments demonstrate that this indicates that less than 0.1 wt% of the triclosan embedded in the plaque have leached out. However, fluoride contained in the plaques does leach out, probably due to the smaller size of the fluoride ions.

The low triclosan leaching was also proven by a different experiment. Plaques as described above and after thermocycling showed a weight difference of +1% (absorption of some water) and not the loss of 6.25% to be expected if all the triclosan had leached out.

The experiments measuring the triclosan leaching of plaques were repeated with a mixture containing a significantly higher triclosan content (40 wt%). Again, plaques were made from a mixture of the varnish components except for the solvent ethanol. These plaques were light-cured and stored in artificial saliva (Ringer solution) for 14 days at 37°C. By UV/Vis spectroscopy, some triclosan could be found in the artificial saliva. Calibration showed that this corresponded to a leaching of only 0.2 wt% of the overall triclosan content of the plaque.

### Mechanical properties

To demonstrate the effect of triclosan on the hardness of the varnish, plaques of approximately 1.2 g (width 2 mm, diameter 25 mm) with varying triclosan contents were made from a mixture of the varnish components except for the solvent. The plaques were light-cured, and Barcol hardness was measured.

The hardness of the antimicrobial varnish containing low triclosan concentrations was found to be as high as the hardness of the varnish not containing any triclosan. Only at higher triclosan concentrations did the hardness of the varnish drop. Thermocycling (500 cycles, 20 seconds at 5°C, 20 seconds at 55°C) does lower hardness somewhat, but not significantly more than with the formulation not containing any triclosan.

### Test D ("<" is "less than")

**Table I**

| (hardness of cured resin formulations; Barcol hardness 934-1) | | | |
|---|---|---|---|
| **Formulation** | **Triclosan** **(wt%*)** | **Resin base** | **Hardness before/ after thermocycling** |
| KP-2-15-2 | - | | 50/46 |
| | | | |
| KP2-35-1 | 10 | KP2-15-2 | 43/38 |
| KP2-35-2 | 15 | KP2-15-2 | 38/32 |
| KP2-35-3 | 20 | KP2-15-2 | 36/31 |
| KP2-35-4 | 25 | KP2-15-2 | 27/23 |
| KP2-35-5 | 30 | KP2-15-2 | 12/<10 |
| KP2-35-6 | 40 | KP2-15-2 | <10/<10 |
| | | | |
| KP2-55 | - | KP2-55 | 40.7 ± 0.7/- |
| BEH1-1-5 | 4 | KP2-55 | 41.5 ± 0-7/- |
| BEH1-1-4 | 6 | KP2-55 | 39.5 ± 1.6/- |
| BEH1-1-3 | 8 | KP2-55 | 39.0 ± 1.5 /- |
| BEH1-1-2 | 10 | KP2-55 | 35.7 ± 0.8/- |
| BEH1-1-1 | 15 | KP2-55 | 35.4 ± 1.7/- |

| | | | |
|---|---|---|---|
| * based on resin mixture | | | |

It has been found that the Barcol hardness of the inventive antimicrobial protective varnish compares favorably to other materials.

| | |
|---|---|
| Cervitec Antimicrobial Varnish (Vivadent, Schaan; an antimicrobial varnish for dentin) | <10 |
| | |
| Delton Pit and Fissure Sealant, light-cured, clear (Dentsply International Inc.; a pit and fissure sealant for enamel). | 38.9±1.5 |

### Example 2: Protective Varnish Containing Nanofiller

A protective varnish for exposed dentin was prepared containing the following components:
- 80 wt% acetone
- 10.5 wt% UDMA-resin (2,7,7,9,15-pentamethyl-4, 13-dioxo-3, 14-dioxa-5, 12-diaza-hexadecan-1,16-diyldimethacrylate)
- 4.8 wt% PENTA (dipentaerythritol pentaacrylate monophosphate)
- 3.0 wt% urethane resin R5-62-1 (7,7,9,63,63,65-Hexamethyl-4, 13,60,69-tetraaza-doheptaconta-1,72-diyldimethacrylate)
- 0.6 wt% ethyl 4-dimethylaminobenzoate
- 0.1 wt% 2,6-di-tert-butyl-p-cresol
- 0.2 wt% cetylamine hydrofluoride
- 0.6 wt% trimethylolpropane trimethacrylate
- 0.2 wt% camphorquinone

To this mixture (100% wt), nanofiller was added (synthesis: see Example 4 below).

This varnish has a low viscosity and deeply penetrates the dentin. After application, the acetone solvent is removed by air-drying. Curing is done with a dental curing lamp with visible light for 20 seconds. A thin, strong polymeric film (thickness approximately 2-6 um) remains.

To demonstrate the effect of nanofiller on the hardness of the varnish, plaques of approximately 1.2 g (width 2 mm, diameter 25 mm) with varying nanofiller contents were made from a mixture of the varnish components except for the solvent. The plaques were light-cured, and Barcol hardness was measured.

The hardness of the varnish containing nanofiller was found to be higher as the hardness of the varnish not containing nanofiller.

### Test E

**Table II**

| Barcol hardness of resin mixtures containing nanofiller glass | | | | |
|---|---|---|---|---|
| **Code Mixture** | **Nanofiller Resin** | **Code Nanofiller** | **Nanofiller** **(wt%*)** | **Barcol Hardness** |
| | KP2-55 | - | - | 40.7 ± 0.7 |
| | BEH1-4-1 | - | - | 41.0 ± 2.2 |
| | BEH1-4-1 | | | 42.4 ± 1.8 |
| | | | | |
| KP2-121-1 | KP2-55 | KP2-121-1 | 5 | 46.5 ± 1.2 |
| KP2-121-2 | KP2-55 | KP2-121-2 | 5 | 43.6 ± 1.6 |
| KP2-123-1 | KP2-55 | KP2-123-1 | 5 | 45.6 ± 1.7 |
| KP2-123-2 | KP2-55 | KP2-123-2 | 5 | 46.9 ± 3.3 |
| KP2-126-1 | KP2-55 | KP2-126-1 | 5 | 46.4 ± 0.8 |
| KP2-126-2 | KP2-55 | KP2-126-2 | 5 | 44.8 ± 1.6 |
| KP2-128-1 | KP2-55 | KP2-128-1 | 5 | 44.8 ± 1.5 |
| KP2-128-2 | KP2-55 | KP2-128-2 | 5 | 45.4 ± 1.7 |
| BEH1-14-1 | BEH1-4-1 | KP2-131-1 | 5 | 44.5 ± 1.6 |
| BEH1-14-2 | BEH1-4-1 | KP2-131-2 | 5 | 45.6 ± 2.4 |
| | | | | |
| BEH1-31-3 | BEH1-4-1 | KP2-131-1 | 7 | 46.3 ± 1.6 |
| BEH1-31-4 | BEH1-4-1 | KP2-131-1 | 8 | 45.9 ± 1.1 |
| BEH1-31-5 | BEH1-4-1 | KP2-131-1 | 9 | 46.7 ± 1.4 |

| | | | | |
|---|---|---|---|---|
| * based on resin | | | | |

The incorporation of nanofiller glass into the varnish formulation clearly increases the hardness of the cured polymer.

A usable varnish formulation preferably has a low viscosity to be capable of sufficiently penetrating the dentin. Any filler incorporated into the varnish formulation therefore, should form a stable sol in the low-viscosity varnish. By "sol" it is meant a highly dispersed solid phase in a liquid phase, the mixture being stable regarding phase separation. A preferred viscosity is below about 1 Pas.

One method of preparing the sol formation, is to mix filler and varnish solution and to put the mixture in an ultrasonic bath for 30 min. For a varnish formulation prepared this way from filler KP2-131-1 and varnish solution, a stability of >3 months has been shown (filler concentration was 1 wt%, composition as mentioned above).

### Example 3: Antimicrobial Protective Varnish Containing Nanofiller and Triclosan

An antimicrobial protective varnish for exposed dentin was prepared containing the following components:
- 80 wt% acetone
- 10.5 wt% UDMA-resin (2,7,7,9,15-pentamethyl-4, 13-dioxo-3, 14-dioxa-5, 12- diaza-hexadecan-1, 16-diyldimethacrylate)
- 4.8 wt% PENTA (dipentaerythritol pentaacrylace monophosphate)
- 3.0 wt% urethane resin R5-62-1 (7,7,9,63,63,65-Hexamethyl-4,13,60,69- tetraoxo-3,14,19,24,29,34,39,44,49,54,59,70-dodecanoxa-5,12,61,68- tetraaza-doheptaconta-1,72-diyldimethacrylate)
- 0.6 wt% ethyl 4-dimethylaminobenzoate
- 0.1 wt% 2,6-di-tert-butyl-p-cresol
- 0.2 wt% cetylamine hydrofluoride
- 0.6 wt% trimethylolpropane trimethacrylate
- 0.2 wt% camphorquinone

To this mixture (100 wt%), nanofiller glass (see Example 4) and triclosan were added in various concentrations.

This varnish has a low viscosity and deeply penetrates the dentin. After application, the ethanol solvent is removed by air-drying. Curing is done with dental curing lamp with visible light for 20 seconds. A thin, strong polymeric film (thickness approximately 2-6 um) remains.

To demonstrate the effects of a combination of a nanofiller and triclosan on the hardness of the varnish, plaques of approximately 1.2 g (width 2 mm, diameter 25 mm) with varying nanofiller/ triclosan contents were made from a mixture of the varnish components except for the solvent. Different mixture ratios of the resins were used. The plaques were light-cured, and Barcol hardness was measured.

**Tab:**

| Hardness of antimicrobial protective varnish formulations containing nanofiller (KP2-123-1) and triclosan | | | | |
|---|---|---|---|---|
| **Code** | **Content Nanofiller** **(wt%*)** | **Content Triclosan** **(wt%*)** | **Resin matrix** **(Code)** | **Hardness Barcol 934-1** |
| BEH1-4-1 | - | - | BEH1-4-1 | 44.1 ± 1.3 |
| BEH1-57-1 | - | 5 | BEH1-4-1 | 42.3 ± 1.4 |
| BEH1-57-2 | 5 | 5 | BEH1-4-1 | 43.4 ± 1.5 |
| BEH1-57-3 | 10 | 5 | BEH1-4-1 | 47.2 ± 2.0 |
| | | | | |
| BEH1-4-2 | - | - | BEH1-4-2 | 54.3 ± 0.8 |
| BEH1-57-4 | - | 5 | BEH1-4-2 | 54.3 ± 1.3 |
| BEH1-57-5 | 5 | 5 | BEH1-4-2 | 54.3 ± 1.3 |
| BEH1-57-6 | 10 | 5 | BEH1-4-2 | 57.8 ± 2.4 |

| | | | | |
|---|---|---|---|---|
| *based on resin | | | | |

BEH1-4-2 is a varnish formulation containing more crosslinker than BEH1-4-1; the basic composition (without triclosan and nanofiller, but with solvent) is
- 80 wt% acetone
- 10.5 wt% UDMA-resin (2,7,7,9,15-pentamethyl-4, 13-dioxo-3, 14-dioxa-5, 12- diaza-hexadecan-1, 16-diyldimethacrylate)
- 4.8 wt% PENTA (dipentaerythritol pentaacrylate monophosphate)
- 3.6 wt% trimethylolpropane trimethacrylate
- 0.6 wt% ethyl 4-dimethylaminobenzoate
- 0.1 wt% 2,6-di-tert-butyl-p-cresol
- 0.2 wt% cetylamine hydrofluoride
- 0.2 wt% camphorquinone

The table shows that low triclosan contents do not affect hardness and that addition of nanofiller leads to a harder varnish.

### Example 4: Synthesis of Nanofiller

Incorporation of fillers into the varnish formulation requires the synthesis of special material. The filler has to be capable of forming a stable sol with the low-viscosity varnish solution to avoid settling of the filler.

In the present invention, Aerosil 380 silanated in an organic solvent is used as nanofiller. It could be shown to form stable sols with the varnish solution after ultrasonic treatment.

Aerosil 380 is a Degussa silica with a BET surface area of 380 m²/g, a primary particle size of 7 nm and 2-3.3 OH groups/nm². Calculating with 2.7 OH groups/nm², this corresponds to 1.7 mmol OH/g Aerosil 380.

A large number of silanated Aerosil 380 fillers were synthesized. The synthesis of a number of fillers is described below.

### KP2-121-1:

8 g Aerosil 380 (undried) and 1.19 g 3-methacryloxypropyl-trichlorosilane were refluxed in 135 g toluene (dried over molecular sieve) for 15 h. The reaction product was dried. The amount of silane employed corresponds to a silylation of approximately 100% of surface OH groups.

### KP2-121-2:

8g Aerosil 380 (undried) and 3.56 g 3-methacryloxypropyl-trichlorosilane were refluxed in 135 g toluene (dried over molecular sieve) for 15h. The reaction product was dried. The amount of silane employed corresponds to a silylation of approximately 300% of surface OH groups.

### KP2-123-1:

8 g Aerosil 380 (undried) and 1.64 g 3-methacryloxypropylmethyl-dichlorosilane were refluxed in 135 g toluene (dried over molecular sieve) for 15h. The reaction product was dried.

### KP2-123-2:

8 g Aerosil 380 (undried) and 3.28 g 3-methacryloxypropylmethyl-dichlorosilane were refluxed in 135 g toluene (dried over molecular sieve) for 15 h. The reaction product was dried.

### KP2-126-1:

8 g Aerosil 380 (dried for 4 d at 120°C) and 1.19 g 3-methacryloxypropyl-trichlorosilane were refluxed in 135 g toluene (dried over molecular sieve) for 15 h. The reaction product was dried.

### KP2-126-3:

8 g Aerosil 380 (dried for 4 d at 120°C) and 3.56 g 3-methacryloxypropyl-trichlorosilane were refluxed in 135 g toluene (dried over molecular sieve) for 15 h. The reaction product was dried.

### KP2-128-1:

8 g Aerosil 380 (undried) and 1.64 g 3-methacryloxypropylmethyl-dichlorosilane were refluxed in 135 g toluene (dried over molecular sieve) for 15 h. The reaction product was dried.

KP2-128-2, KP2-131-1 and KP2-131-2 were synthesized accordingly. The silanes employed are listed in the table below.

To control the degree of silanation of the Aerosil 380, a simple hydrophobicity test was carried out. The silanated Aerosil was powdered, and a smooth surface was created by applying pressure to the material with a glass plate. A drop of water was placed on top of the smooth surface, and the time until vanishing of the drop of water was measured. This method allows a rough comparison of hydrophobicity as with more hydrophilic materials, the water penetrates them more rapidly.

**Tab:**

| hydrophobic behavior of Aerosil 380 silanated with different agents and ratios (M:3-methacryloxypropyl): the time a drop of water needed to penetrate into the material was measured (long penetration time = hydrophobic material). | | | | |
|---|---|---|---|---|
| **Code** | **Silane** | **ratio CI/OH** | **Pretreatment of Aerosil** | **Penetration time (h)** |
| KP2-121-1 | MSiCl₃ | 1:1 | undried | 0 |
| KP2-121-2 | MSiCl₂ | 3:1 | undried | 5 |
| KP2-123-1 | MSiMeCl₂ | 1:1 | undried | 1 |
| KP2-123-2 | MSiMeCl₂ | 2:1 | undried | >5 |
| KP2-126-1 | MSiCl₃ | 1:1 | dried | 0 |
| KP2-126-2 | MSiCl₃ | 3:1 | dried | 1 |
| KP2-128-1 | MSiMeCl₂ | 1:1 | dried | 1.5 |
| KP2-128-2 | MSiMeCl₂ | 2:1 | dried | 3 |
| KP2-131-1 | MSiMe₂Cl | 1.3:1 | dried | 4 |
| KP2-131-2 | MSi(OMe)₃ | 4.5:1* | dried | 0 |

| | | | | |
|---|---|---|---|---|
| * (OMe/OH) | | | | |

1% of these nanofillers was suspended in a mixture of 81 wt% acetone, 13 wt% UDMA (urethane dimethacrylate) and 6 wt% PENTA (dipentaerythritol pentaacrylate monophosphate). The mixtures were put into an ultrasonic bath for 3 hours. The mixtures were then left undisturbed. After 3 hours, the suspensions were checked for settling of material.

No settling of filler was observed using the fillers KP2-121-1, KP2-12301, KP2-123-2, KP2-128-1, KP2-131-1.

Very little settling of filler was observed with fillers KP2-126-1 and KP2-128-2.

Little settling was observed with fillers KP2-121-2 and KP2-126-2.

Some settling was observed with filler KP2-131-2.

The mixture with KP2-131-1 was left undisturbed for 1 month. Only very little settling of filler could be observed. This small amount of filler could easily be resuspended by shaking the mixture.

The results show that by silanation of Aerosil 380 in toluene, it is possible to obtain a hydrophobic functionalized nanofiller if an excess of silane is used.

Silanation can also be proven by IR spectroscopy as the methacrylate group of the silane displays a strong carbonyl peak.

The foregoing description illustrates preferred embodiments of the invention. However, concepts employed may, based upon such description, be employed in other embodiments without departing from the scope of the invention. Accordingly, the following claims are intended to recite the invention broadly, as well as in the specific forms herein.

## Claims

1. A protective varnish that strengthens exposed dentin, comprising
a) at least one light curable polymerizable monomer
selected from the group consisting of methacrylate and acrylate monomers having at least one unsaturated double bond, and mixtures thereof,
in a solvent
selected from the group consisting of water, acetone, ethanol, ethyl acetate, other organic solvents with boiling points below that of water and mixtures thereof,
b) 0,001 to 20 wt.-% of 2,4,4'- trichloro-2'-hydroxydiphenyl ether , based on 100 wt.-% of the varnish, and
c) a filler.

2. A varnish as in claim 1, having a viscosity of from about 0.0001 to about 1 Pas.

3. A protective varnish as set forth in claim 1, further comprising at least one additive component selected from the group consisting of dental resins, fluoride, stabilizers, initiators, and solvents.

4. A protective varnish as in claim 3, wherein said filler forms stable sols and the varnish having a viscosity below about 1 Pas.

5. A protective varnish as in claim 3, wherein said filler is selected from the group consisting of ground glass, ground quartz, highly dispersed silica, zeolite, laponite, kaolinite, vermiculite, mica, ceramic metal oxides, alumina, pyrogenic silica, sparingly volatile oxides of titanium, zirconium, germanium, tin, zinc, iron, chromium, vanadium, tantalum, niobium, and mixtures thereof.

6. A protective varnish as set forth in claim 3, wherein the filler is treated with an agent enabling said filler to form a stable sol in a solvent mixture having a viscosity below about 1 Pas.

7. A protective varnish as set forth in claim 6, wherein said agent is a silanating agent.

8. A protective varnish as set forth in claim 7, wherein said silanating agent has at least one polymerizable double bond and at least one group that easily hydrolyses with water.

9. A protective varnish as set forth in claim 7, wherein said silanating agent is selected from the group of 3-methacryloxypropyltrimethoxysilane, 3-methacryloxypropyldimethoxy-monochlorosilane, 3-methacryloxypropyldichloromonomethoxylsilane, 3-methacryloxypropyltrichlorosilane, 3-methacryloxypropyldichloromonomethylsilane, 3-methacryloxypropylmonochlorodimethylsilane, and mixtures thereof.

10. A method of preparing a protective varnish for exposed dentin as defined by any one of claims 1 to 9, said method comprising the step of forming a solution of the antimicrobial agent and the at least one polymerizable monomer in the solvent, and further comprising mixing a filler material into said solution.

11. A method as in claim 10, wherein said filler material is pretreated with a silanating agent in an organic solvent.

## Patentansprüche

1. Schutzlack, der freigelegtes Dentin festigt, umfassend
a) mindestens ein lichthärtbares, polymerisierbares Monomer,
ausgewählt aus der Gruppe bestehend aus Methacrylat- und AcrylatMonomeren mit mindestens einer ungesättigten Doppelbindung und deren Mischungen
in einem Lösungsmittel,
ausgewählt aus der Gruppe bestehend aus Wasser, Aceton, Ethanol, Ethylacetat, anderen organischen lösungsmitteln mit Siedepunkten unter dem von Wasser und deren Mischungen,
b) 0,001 bis 20 Gew.-% von 2,4,4'-Trichlor-2'-hydroxydiphenylether, bezogen auf 100 Gew.-% des Lackes und
c) einen Füllstoff.

2. Lack nach Anspruch 1 mit einer Viskosität von etwa 0,0001 bis etwa 1 Pa·s.

3. Schutzlack nach Anspruch 1, weiter umfassend mindestens eine Zusatz-Komponente, ausgewählt aus der Gruppe bestehend aus Dentalharzen, Fluorid, Stabilisatoren, Initiatoren und Lösungsmitteln.

4. Schutzlack nach Anspruch 3, worin der Füllstoff stabile Sole bildet und der Lack eine Viskosität unter etwa 1 Pa·s aufweist.

5. Schutzlack nach Anspruch 3, worin der Füllstoff ausgewählt ist aus der Gruppe bestehend aus gemahlenem Glas, gemahlenem Quarz, stark dispergiertem Siliciumdioxid, Zeolith, Laponit, Kaolinit, Vermiculit, Glimmer, keramischen Metalloxiden, Aluminiumoxid, pyrogenem Siliciumdioxid, kaum flüchtigen Oxiden von Titan, Zirkonium, Germanium, Zinn, Zink, Eisen, Chrom, Vanadium, Tantal, Niob und deren Mischungen.

6. Schutzlack nach Anspruch 3, worin der Füllstoff mit einem Mittel behandelt ist, das es dem Füllstoff ermöglicht, ein stabiles Sol in einer Lösungsmittelmischung mit einer Viskosität unter etwa 1 Pa·s zu bilden.

7. Schutzlack nach Anspruch 6, worin das genannte Mittel ein Silanierungs-Mittel ist.

8. Schutzlack nach Anspruch 7, worin das Silanierungs-Mittel mindestens eine polymerisierbare Doppelbindung und mindestens eine Gruppe aufweist, die leicht mit Wasser hydrolysiert.

9. Schutzlack nach Anspruch 7, worin das Silanierungs-Mittel ausgewählt ist aus der Gruppe von 3-Methacryloxypropyltrimethoxysilan, 3-Methacryloxypropyldimethoxymonochlorsilan, 3-Methacryloxypropyldichlormonomethoxysilan, 3-Methacryloxypropyltrichlorsilan, 3-Methacryloxypropyldichlormonomethylsilan, 3-Methacryloxypropylmonochlordimethylsilan und deren Mischungen.

10. Verfahren zum Herstellen eines Schutzlackes für freigelegtes Dentin, wie nach einem der Ansprüche 1 bis 9 definiert, wobei das Verfahren die Stufe des Bildens einer lösung des antimikrobiellen Mittels und des mindestens einen polymerisierbaren Monomers im Lösungsmittel und weiter das Einmischen eines Füllstoff-Materials in die Lösung umfasst.

11. Verfahren nach Anspruch 10, worin das Füllstoff-Material mit einem Silanierungs-Mittel in einem organischen Lösungsmittel vorbehandelt ist.

## Revendications

1. Vernis protecteur qui renforce la dentine exposée, comprenant :
a) au moins un monomère polymérisable photo-durcissable
choisi dans le groupe consistant en des monomères méthacrylate et acrylate ayant au moins une double liaison insaturée, ainsi que leurs mélanges,
dans un solvant
choisi dans le groupe consistant en l'eau, l'acétone, l'éthanol, l'acétate d'éthyle, d'autres solvants organiques ayant des points d'ébullition inférieurs à celui de l'eau, ainsi que leurs mélanges,
b) 0,001 à 20 % en poids d'éther de 2,4,4'-trichloro-2'-hydroxydiphényle, sur la base de 100 % en poids du vernis, et
c) une charge.

2. Vernis suivant la revendication 1, ayant une viscosité d'environ 0,0001 à environ 1 Pa.s.

3. Vernis protecteur suivant la revendication 1, comprenant en outre au moins un constituant additif choisi dans le groupe consistant en des résines dentaires, un fluorure, des stabilisants, des initiateurs et des solvants.

4. Vernis protecteur suivant la revendication 3, dans lequel ladite charge forme des sols stables, le vernis ayant une viscosité inférieure à environ 1 Pa.s.

5. Vernis protecteur suivant la revendication 3, dans lequel ladite charge est choisie dans le groupe consistant en le verre broyé, le quartz broyé, la silice hautement dispersée, la zéolite, la laponite, la kaolinite, la vermiculite, le mica, des oxydes métalliques céramiques, l'alumine, la silice pyrogène, des oxydes faiblement volatils de titane, de zirconium, de germanium, d'étain, de zinc, de fer, de chrome, de vanadium, de tantale, de niobium et leurs mélanges.

6. Vernis protecteur suivant la revendication 3, dans lequel la charge est traitée avec un agent permettant à ladite charge de former un sol stable dans un mélange de solvants ayant une viscosité inférieure à environ 1 Pa.s.

7. Vernis protecteur suivant la revendication 6, dans lequel ledit agent est un agent de silanation.

8. Vernis protecteur suivant la revendication 7, dans lequel ledit agent de silanation possède au moins une double liaison polymérisable et au moins un groupe qui subit aisément une hydrolyse avec de l'eau.

9. Vernis protecteur suivant la revendication 7, dans lequel ledit agent de silanation est choisi dans le groupe consistant en 3-méthacryloxypropyltriméthoxysilane, 3-méthacryloxypropyldiméthoxymonochlorosilane, 3-méthacryloxypropyldichloromonométhoxysilane, 3-méthacryloxypropyltrichlorosilane, 3-méthacryloxypropyldichloromonométhylsilane, 3-méthacryloxypropylmonochlorodiméthylsilane et leurs mélanges.

10. Procédé pour la préparation d'un vernis protecteur pour de la dentine exposée, suivant l'une quelconque des revendications 1 à 9, ledit procédé comprenant l'étape consistant à former une solution de l'agent antimicrobien et dudit au moins un monomère polymérisable dans le solvant, et comprenant en outre le mélange d'une charge à ladite solution.

11. Procédé suivant la revendication 10, dans lequel ladite charge est prétraitée avec un agent de silanation dans un solvant organique.
